# EUROPEAN PATENT SPECIFICATION

(11) **EP 1 287 834 B1**
(45) Date of publication and mention of the grant of the patent: **02.06.2004**
(21) Application number: 02019045.0
(22) Date of filing: 27.08.2002
(51) Int. Cl.: A61L 2/18, A61L 2/20, C01B 13/10

(54) **Method of ozone sanitation**
Ozonsterilisationsverfahren
Mèthode de la stérilisation avec ozone

(30) Priority: 29.08.2001 US 940489
(43) Date of publication of application: 05.03.2003
(73) Proprietor: PRAXAIR TECHNOLOGY, INC., Danbury, CT 06810-5113 (US)
(72) Inventor: Bon, Paulo Sergio, Sao Paulo 06642-210 (BR); Ribeiro, Sergio Roberto Rodrigues, 80 Barra de Tijuca, Rio de Janeiro, RJ (BR); Pareja, Henrique Armando Langaro, Rio de Janeiro, RJ (BR)
(74) Representative: Schwan - Schwan - Schorer

(56) References cited:
- US-A- 2 970 821
- US-A- 5 368 815
- US-A- 5 484 549
- PATENT ABSTRACTS OF JAPAN vol. 1995, no. 11, 26 December 1995 (1995-12-26) & JP 07 213591 A (MASAYUKI EZAWA), 15 August 1995 (1995-08-15)

## Description

### Field of the Invention

The present invention relates to sanitizing the surfaces of solid articles with an ozone solution.

### Background of the Invention

All processing industries that prepare products which are susceptible to damage by microbial contamination must from time to time sanitize the equipment that is used. Examples of such industries include the food, pharmaceutical, brewing and wine industries. Examples of equipment that must be sanitized include storage tanks, pumps, mixing tanks, coolers, carbonators, fillers, filling lines and the like, as well as containers such as bottles and cans.

**US-A-5 484 549** discloses a pH greater than 7 aqueous ozonized cleaning composition that can be used for cleaning a tenacious residue or film from solid surfaces. Immediately after ozone generation, the ozone containing gas stream comprising ozone and the residual air is injected through a hose into an aqueous potentiating additive containing carrier solution, forming a cleaning solution which is applied immediately to a soiled solid surface to remove a contaminating residue or film.

There are several problems faced by such industries during conventional sanitation activities. Some of these problems are the following.

Sanitation time interrupts production. So, there is a strong customer need to reduce, as much as possible, the sanitation cycle time.

Sanitation imposes capital and operating costs. There is always a need to reduce costs. There are two conventional sanitation processes. The first one is called a hot process, which applies hot water or water steam as the sanitizing agent. This alternative imposes capital costs related to having a boiler, plus operating costs related to power or fuel consumption. The second one is called a cold process, which applies chemicals such as peracetic acid, caustic soda, chlorine based products or hydrogen peroxide based products, as sanitizing agent. This alternative imposes capital costs related to application equipment, and operating cost related to the chemicals.

Sanitation imposes maintenance costs. Hot water and conventional chemicals cause significant and frequent damage to equipment parts, such as gaskets, o-rings, metal pieces and others, which consequently require frequent maintenance.

Chemical agents such as peracetic acid or peroxide based products are quite expensive when compared to other alternatives, and are also aggressive to the materials frequently used in beverage processing equipment (such as sealants).

Chemical agents based on chlorine are usually aggressive to stainless steel (pit corrosion) after a large number of sanitation cycles, which consequently facilitates the accumulation of soil and hence multiplication of microorganisms. It is important to remember that all filling lines and other processing equipment in beverage and food industries are made of stainless steel. Chlorine based products are usually cheap, which makes their use attractive, but they require a longer contact time than alternative sanitation chemicals, which increases the tendency to corrosive attack.

Hot sanitation applies high temperature as the sanitizing agent. It is known that high temperature always accelerates damage to equipment, requiring more frequent maintenance service - preventive and corrective - which consequently increases maintenance costs.

Sanitation imposes safety concerns. A strong concern related to hot sanitation processes is the explosion risk associated with a possible mechanical or operational failure. Most beverage products are cooled during processing and the preferred cooling agent is generally ammonia, due to its cost-effectiveness and technical advantages. It is not uncommon that due to an operational or mechanical failure, hot water would contact the ammonia, causing an unexpected heating and consequently creating a hazardous condition with an explosion risk. In addition, a very important aspect of hot sanitation processes is the risk associated with the high temperature. Unexpected leakage, direct contact of the hot water with skin, eyes, etc., is always a concern.

In cold processes, safety aspects related to handling and inhalation of chemicals (volatiles, concentrates, solutions, etc.) are always a strong concern.

Sanitation consumes water. All conventional sanitation processes need large amounts of water. As a reference, the beverage industry usually consumes an average of 2.5 liters of water per liter of product. This imposes costs and imposes a burden on the feasibility of operating in geographic regions where adequate supplies of water may not always be available.

Sanitation processes are always challenged to achieve adequate microbiological control. Due to the organic matter content characteristic in beverage and food products, it is absolutely natural to have significant microbiological growth inside storage tanks and processing equipment. So, sanitation activities are needed for maintaining product quality.

The objective of any sanitation process is to reduce the presence of microorganisms down to acceptable levels in processing equipment or in beverage or food containers, or even to eliminate microorganisms, so that it is possible to assure the expected product quality. Acceptable levels of microbiological contamination are usually determined by company standards or local regulations.

Prior sanitation processes have involved three types of practice; hot sanitation; cold sanitation, based on chemicals; and ozone sanitation.

Hot sanitation is based on the temperature of hot water or water steam as sanitizing agent. Working with temperatures in the range of 90°C and 100°C and, keeping a minimum contact time, it is possible to reduce considerably the presence of microorganisms. All sanitation processes using temperature as the active agent, need a heat source and a heat exchanger system to heat.the water or to produce water steam. The most common system used for this kind of application is a boiler, which usually consumes fuel or electric power. Hot sanitation processes generally work well in solving microbiological challenges.

In terms of safety risks related to high temperature, a reliable monitoring and control system is required to avoid or minimize such risks. The most common alternative to minimize risks related to high temperature is to use a combined system, applying a cold process only for sanitizing the carbocooler (where the ammonia is located) in series with the hot process applied for the rest of the line. This solution increases significantly the time required for a complete sanitation cycle. It is also possible to apply both processes partially in parallel. However, this is an expensive solution and the sanitation time is still much longer than a cold process based on chemicals or even ozone. Maintenance costs are always a problem in hot sanitation.

Alternatively, it is possible to consider a system that removes all the ammonia from the cooler during the hot sanitation process, but this is very expensive and demands a significant amount of time to remove the ammonia from the cooler volume. However, sanitation time means stopped production, and consequently lower productivity.

Operating costs related to hot sanitation are usually lower than cold processes based on peracetic acid (see cold sanitation) and higher than chlorine based processes, as well as ozone based alternatives.

Cold sanitation is based on a chemical agent for eliminating or reducing microorganisms. Useful chemical agents include peracetic acid, chlorine based products and peroxide (less common) based products. In all of these alternatives, the risks associated with explosion no longer exist, since the entire process is applied at ambient temperature.

Chemical agents such as peracetic acid or peroxide based products are quite expensive when compared to other alternatives. Such products are also aggressive to the most usual materials used on beverage processing equipment (sealant and others). Peracetic acid works well in microbiological control. Contact time when applying peracetic acid is the shortest of the conventional alternatives. Handling is always a very strong concern.

Existing ozone sanitation processing, which applies ozone as the sanitation agent, has conventionally been based on systems that dissolve ozone in water through a by-pass at the process line plus an ozonated water recycle tank, recirculating water from and back to the tank (as seen in US-A-5,368,815). In this way, a portion of the water pumped to the process is then diverted to the by-pass line and then back to the ozonated water recycle tank. A commercial design Venturi entrains ozone that is partially dissolved by the Venturi and partially dissolved by the water column in the tank. Although conventional implementation of an ozone process solves the problems related to microbiology and water consumption, it does not satisfactorily solve problems related to safety, maintenance and sanitation costs.

### Brief Summary of the Invention

One aspect of the present invention is a method for sanitizing, comprising
(A) preparing an aqueous solution of ozone by
   (A.1) manufacturing a gaseous stream of ozone from oxygen having a purity of at least 90 vol.%,
   (A.2) providing a stream of water having a pH of 6.5 - 7.5 from a source which monitors the pH of said water and adjusts the pH as necessary to maintain the pH in said range, and
   (A.3) injecting said gaseous stream of ozone directly into said stream of water;
(B) feeding said aqueous solution of ozone into a tank which holds said solution and which has over said solution a gaseous atmosphere comprising ozone;
(C) applying said aqueous solution of ozone from said tank onto a surface to be sanitized; and
(D) recovering water from said surface and recycling it to said source.

### Brief Description of the Drawings

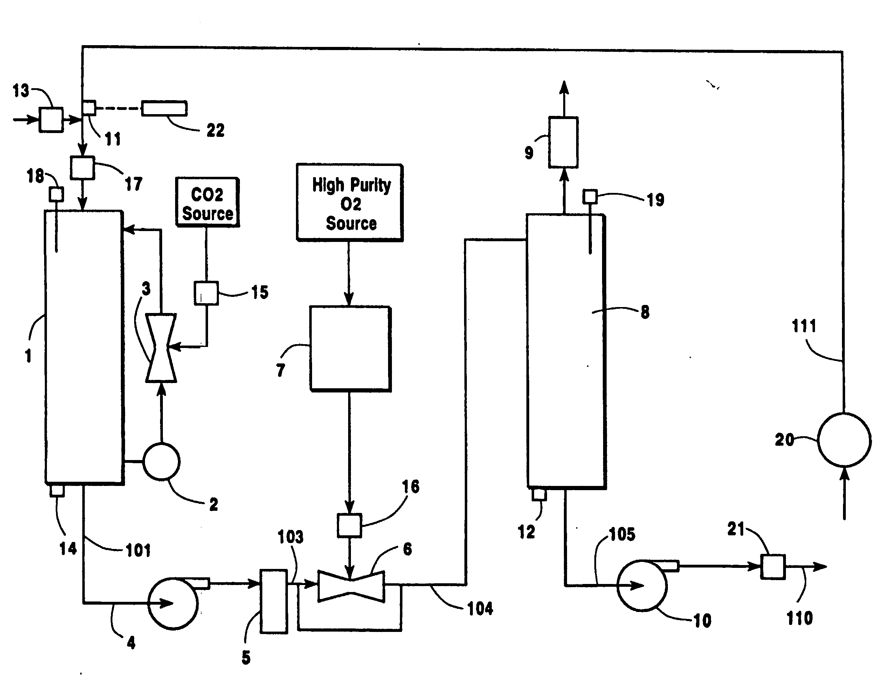

Figure 1 is a flowsheet showing an embodiment of the present invention.

### Detailed Description of the Invention

Figure 1 depicts the components of an embodiment of the invention. It should be understood, though, that these components can conveniently be mounted on a skid or other means for providing the components on-site to a customer in a form ready to utilize in sanitizing an article or apparatus.

The apparatus is assembled on a frame skid and comprised of the following main components:
- Water storage tank (1)
- pH system pump (2)
- O₃ Ejector (3)
- Pump for filtering and O₃ dissolution (4)
- Filter (5)
- Praxair Ejector (6)
- Ozone generator (7)
- Gas-Liquid phase separator (8)
- Ozone destroyer (9)
- Process pump (10)
- Dissolved Ozone Sensor (11)
- Dissolved ozone Monitor (22)
- Dissolved ozone Sensor (12)
- Dissolved ozone monitor (23)

Water tank 1 stores water to be used for sanitizing. The volume of water should be at least enough to sanitize the intended object or objects. Typically the tank volume is 1 m³ to 5 m³. When needed, an amount of makeup water is fed by opening the valve 13.

The pH of the water in tank 1 is continually monitored and automatically controlled so that it the pH is in the range of 6.5 to 7.5. For instance, if pH sensor 14, in tank 1, indicates a pH higher than 7.5, the monitor conveys a signal to turn on pump 2 and open valve 15, thereby entraining an amount of carbon dioxide via injector 3 (or other acidic material, such as sulfuric acid) that is sufficient to lower the pH to within the range of 6.5 - 7.5 (such as 7.0). Then, the sensor signals pump 2 to turn off and valve 15 to close. Conversely, if the pH of the water in tank 1 falls below 6.5, a pH sensor activates addition of alkaline material such as a sodium hydroxide solution until the pH rises to a value between 6.5 and 7.5, and then closes the valve and discontinues addition of the alkaline material.

Pump 4 pumps water out of tank 1 via line 101, and along line 102 through filter 5 and then via line 103 through injector 6. By passing through the filter, solids present in the water are removed from the water and retained in the filter.

Passing through injector 6 injects ozone into the water. The ozone is formed in ozone generator 7, which is fed by any source of high purity, preferably nearly pure, oxygen. Oxygen purity of at least 90 vol.% is preferred, and oxygen purity of at least 99 vol.% is more preferred. Satisfactory sources include liquid oxygen, cylinders or other sources of packaged gas, VPSA or PSA.

The amount of ozone injected by injector 6 should be sufficient to form a solution comprising 1 % (w/w) to 15 % (w/w) in the stream that exits the injector via line 104. An alternative combination of a dissolution device plus a gas-liquid phase separator device can be applied in-line. For instance, conventional Venturi, tubular reactor, bubble contact columns, staged contact chambers can optionally be used for Ozone dissolution. In addition, forced separators such as fan (propeller) based devices, as well as stripping systems based on inert gas bubbling or systems based on vacuum (negative pressure) formation in the separator tank headspace can also be applied alternatively for separation of the non-dissolved gases (mainly oxygen, nitrogen and residual ozone). Most of the ozone gas is dissolved in the water in stream 104 before it reaches the phase separator tank 8.

In tank 8, undissolved gas reports to the vessel headspace while the liquid phase is held in the lower portion of the vessel. A vent is provided through which excess gas can controllably be vented to the atmosphere. The preferred pressure in the headspace is atmospheric. Slightly higher pressures, up to about 1.01 atmospheres, can be permitted.

Before being vented to the atmosphere, any residual ozone gas content in the gas being vented is completely eliminated by an ozone destroyer 9 which is preferably a thermal destroyer but can instead be a chemically-based system. In addition, residual ozone can also be re-entrained into the system, this way optimizing ozone utilization.

Retention time of the solution inside tank 8 is relatively short, but should be long enough for the gas (ozone) and liquid (solution) phases to separate. The dissolved ozone concentration in the solution leaving via line 105 is quite high, generally about 1 ppm to about 10 ppm.

Line 105 passes through pump 10 and valve 21 and terminates at 110, which can be capped or sealed with a valve, or other fitting which facilitates application of ozonated solution to the product or service being sanitized. Pump 10 pumps ozonated water from the tank 8 to the process line. Water is then recaptured from the product to which the ozonated solution was applied, and is driven by recycle pump 20 through return line 111 back to water tank 1.

When sanitation starts, pump 4 is turned on, as are ozone generator 7, pump 10, and valves 17, 16 and 21. The water level inside tank 1 is monitored and controlled by level sensors 18, and the water level inside tank 8 is monitored and controlled by level sensor 19.

The sanitation process is controlled by a dissolved ozone sensor 11 plus a dissolved ozone monitor 22, which are placed in line 111 feeding to tank 1. The data from these is used by a controller (not shown) to control how much ozone is allowed to pass through valve 16 into the stream at injector 6.

Operation is preferably maintained by continual monitoring of ozone concentration over time. That is, for a given operation a desired target aggregate value of (ozone concentration times treatment time) is prescribed. Then, periodically during the sanitation operation, values are tabulated of ozone concentration times time (usually in seconds). When the aggregate sum of these periodically determined values reaches or exceeds the target value, the operation is considered completed and is discontinued.

Preferred aggregate values of concentration-time are 4.0 or higher, measuring the time in minutes and the concentration in parts per million (ppm).

Compared to the aforementioned hot sanitation processes, the present invention provides many advantages including the following:

Time is saved and hence productivity increases. To carry out hot sanitation of a beverage-making operation, there is a carbocooler that must be sanitized by a cold process even if the rest of the equipment (storage tank, piping, filler, mixer, valves, etc.) is sanitized by a hot process. Field tests have shown that time savings on sanitation can be as high as 75% when a hot sanitation process is replaced by the process of the present invention.

Safety risks related to the handling of hot water and steam are eliminated. The present invention applies ozonated water at ambient temperature, so these risks are eliminated.

Maintenance costs are reduced. Ozonated water is less aggressive then high temperature and consequently reduces maintenance requirements.

Explosion risks are eliminated. Beverages are usually cooled by ammonia during the filling operation during which the beverage is filled into bottles. In the case of an operational or mechanical failure during the sanitation process, hot water or water steam might come directly or indirectly into contact with ammonia, presenting a serious explosion risk due to the rapid expansion of the ammonia when it is heated quickly. The ozonated water used in the present invention eliminates such risk.

Operating costs are reduced. Using hot water or steam costs money because of the boilers used to heat the water or to produce water steam, and the fuel required for combustion. The present invention does not need fuels or boilers, which reduces capital cost and operating cost.

Compared to cold sanitation based on chemicals, the present invention provides many advantages including the following.

Safety risks related to the handling of chemicals are eliminated. Handling of chemicals used for sanitation is always a matter of concern, because they can cause serious skin irritation and burns in the case of direct contact, in high or even low concentrations. Breathing of volatile compounds is also a concern. Any failure, mechanical or operational, risks causing serious damage to the operator's health. In the system of the present invention, ozone is generated and dissolved, and residual ozone is destroyed in a unique skid, which is automatically operated, monitored and controlled. So, the risks of direct contact of the ozone gas or solution with the operator is eliminated, or at least much lower than the risk related to conventional chemicals alternatives.

Time is saved and productivity increases. Commercial chemicals used for sanitation generally have to be applied in a concentration between 0.15% and 2%. Lower or even higher concentration levels actually reduce the sanitation capacity or provide no additional effect. Since it is not possible to vary chemical concentration, it is also not possible to reduce contact time below a minimum recommended time for each specific product. In the present invention, as ozone concentration can be varied in a large concentration range, contact time can also be varied for the particular sanitation operation being carried out. As higher ozone concentrations permit shorter contact times, it is possible to save up to 50% of the total cleaning-in-place process time. {As used herein, cleaning-in-place or "CIP" means cleaning and sanitation steps.)

Operating costs are reduced. The operating cost of the system of the present invention is significantly lower than the cold sanitation alternative based on chemicals. Typical comparative cost figures are provided in the examples.

Maintenance costs are reduced. Most of the chemical agents used for sanitation purpose cause severe damage to sealing materials when applied repeatedly, requiring frequent maintenance services. Viton, teflon, buna-N and neoprene are the most usual sealing materials employed. Ozonated water is less aggressive then conventional chemicals and consequently reduces maintenance requirements.

Wastewater treatment needs are reduced. Chemicals used for sanitation purposes are carried out from the process to the wastewater treatment plant and can interfere with the wastewater treatment process. At that particular stage, chemicals are actually contributing as a new pollutant. Since the ozone that is not consumed during the sanitation process itself decomposes safely to oxygen, it is not expected to reach the treatment plant.

Compared to prior systems using ozone, the present invention provides advantages including the following.

Direct injection of ozone into the water, especially if carried out with the apparatus of US-A-4,743,405 which apparatus represents an especially preferred mode of the present invention, provides higher and faster transfer of gaseous ozone into solution.

Higher ozone transfer efficiency is also provided by the high ozone partial pressure in the gas stream that is combined with the water to form the ozone solution. The present invention works with high purity oxygen (at least 90 vol.%, preferably at least 99 vol.% oxygen) as feed source for the ozone generator. This way, the ozone gas production is higher than it would be when air (21% oxygen) is the feed source. Prior processes have used air as the feed source for the ozone generator. The same ozone generator that produces 5% ozone w/w (in weight) when working with air would produce up to 15% ozone w/w, when working with pure oxygen. This means a significant increase in partial pressure of ozone, which consequently allows the present invention to achieve higher dissolution rates, when compared to the prior art, as well as achieve higher ozone dissolution levels.

Higher ozone utilization due is provided by the in-line direct injection of ozone. The present invention dissolves ozone directly inside the line that carries the stream. Since the injector provides higher dissolution rates than the prior art, it is possible to dissolve ozone directly in the same line that needs to be sanitized, i.e., all the dissolved ozone comes immediately into direct contact with the surface of the object that needs to be sanitized. Prior processes dissolve ozone into water through a by-pass line which includes an ozonated water tank such that water is then kept inside that tank, being recirculated through the by-pass line, until the desired ozone concentration is achieved, when the prior art system starts pumping the ozonated water to the articles that need to be sanitized.

Ozone is unstable and consequently a certain portion of the ozone is naturally destroyed before getting coming into contact with the surface to be sanitized, which doesn't occur in the proposed system. Consequently, the proposed system has a higher ozone utilization rate.

Higher ozone utilization is also achieved because of the pH control. The present invention includes pH control that keeps the pH of the water that makes up the ozone solution as close as possible to neutral pH, i.e., in between the range 6.5 to 7.5, which has been found to be the range in which ozone has its highest lifetime in water.

Maintenance and capital costs are reduced. An ozone generator using air as the oxygen source must have a compressor, filter and dryer for preparing and treating the air that is fed to the generator. These additional pieces of equipment are relatively expensive and require frequent maintenance services. An ozone generator based on high purity oxygen does not need such additional equipment, because the oxygen is already dry, compressed and clean. This provides significant savings in maintenance costs.

Energy savings are realized because the high purity oxygen fed to the ozone generator includes significantly less nitrogen and other gases that would otherwise be "dead weight" needing to be transported and compressed.

Residual undissolved ozone is destroyed before leaving the system, contributing to safety and environmental cleanliness. The ozone destroyer placed at the outlet of the headspace of the gas-liquid separator tank assures that all residual undissolved ozone is completely destroyed instead of being vented to the atmosphere. Ozone reaching the ambient atmosphere around the apparatus is extremely hazardous for the operators.

It has also been determined that adequate sanitation can be provided by controlling the operation so that the contact time with the ozone solution is based not on a specified minimum concentration, as has been the prior art practice, but on a predetermined product of concentration (C) with contact time (t).

The previous mode of control would not take into account operation during times when the ozone concentration fell below the given minimum. For instance, in the prior control practice, if the predetermined "C" is 0.5 mg/l and the predetermined "t" is 10 minutes, the system would require a total of 10 minutes of operation at ozone concentration of at least 0.5 mg/l, even if there is an interim period when the ozone concentration is below that minimum.

Using the control scheme preferred for operation of the present invention, all contact time with an ozone solution is taken into account. For instance, assuming a predetermined C*t = 5.0 (obtained by multiplying together the aforementioned 0.5 mg/l and 10 minutes), a complete cycle time could take no more than 10 minutes: periods of lower ozone concentration that would be ignored by the previous control scheme would be included by periodically summing the concentrations times the length of time that the ozone concentration is at that lesser concentration.

### EXAMPLES

### Example 1

This example compares the typical overall costs of sanitizing a syrup storage tank used in a soft drink beverage producer by the present invention and by a typical hot sanitation process.

| | Hot Sanitation Process | Present Invention |
|---|---|---|
| Sanitation Time | 10 minutes | 5 minutes |
| Operating Cost per Run | US$ 13.82 | US$ 1.58 |
| Safety Risk | High | Low |
| Maintenance Cost | Higher | Lower |

These conclusions were based on the assumptions that:
1) sanitation time of 5 minutes in the present invention is based on a C*t of 2.0, i.e., an ozone average concentration = 0.4 ppm and contact time of 5 minutes;
2) sanitation time of 10 minutes in the hot process is based on water temperature of 90°C;
3) operating cost in the hot process is based on boiler powered by natural gas (US$0.18/m³); and
4) operating cost of the present invention is based on an oxygen cost of US $300./ton.

### Example 2

This example compares the same application of the present invention to sanitizing by a conventional chemical sanitation process based on peracetic acid.

| | Chemical (Peracetic) Sanitation Process | Present Invention |
|---|---|---|
| Sanitation Time | 10 minutes | 5 minutes |
| Operating Cost per Run | US$ 22.12 | US$ 1.58 |
| Safety Risk | High | Low |
| Maintenance Cost | Higher | Lower |

These conclusions were based on the assumptions that:
1) sanitation time of 5 minutes in the present invention is based on a C*t of 2.0, i.e., ozone average concentration = 0.4 ppm and contact time of 5 minutes;
2) sanitation time of 10 minutes in the chemical process is based on peracetic acid at 0.15% concentration;
3) operating cost in chemical process is based on peracetic acid at US$ 4.00 / Kg - supplied at 15% concentration; and
4) operating cost of the present invention is based on an oxygen cost of US$ 300/ton.

### Example 3

This example compares the same application of the present invention to sanitizing by a conventional chemical sanitation process based on chlorine.

| | Chemical (Chlorine) Sanitation Process | Present Invention |
|---|---|---|
| Sanitation Time | 20 minutes | 5 minutes |
| Operating Cost per Run | US$ 0.13 per run | US$ 1.58 per run |
| Safety Risk | Low | Low |
| Maintenance Cost | Medium | Lower |

These conclusions were based on the assumptions that:
1) sanitation time of 5 minutes in the present invention is based on a C*t of 2.0, i.e., ozone average concentration = 0.4 ppm and a contact time of 5 minutes;
2) sanitation time of 20 minutes in the chemical process is based on chlorine applied at 50 ppm concentration;
3) operating cost in chemical process is based on chlorine supplied at US$ 0.23 liter - supplied at 9 % concentration;
4) operating cost in the present invention is based on an oxygen cost of US$ 300/ton; and
5) maintenance cost of the chlorine-based process refers to pit corrosion formation in stainless steel AISI-304 (most usual material of piping and other metallic components of beverage and food industries).

## Claims

1. A method for sanitizing, comprising
(A) preparing an aqueous solution of ozone by
(A.1) manufacturing a gaseous stream of ozone from oxygen having a purity of at least 90 vol.%,
(A.2) providing a stream of water having a pH of 6.5 - 7.5 from a source which monitors the pH of said water and adjusts the pH as necessary to maintain the pH in said range, and
(A.3) injecting said gaseous stream of ozone directly into said stream of water;
(B) feeding said aqueous solution of ozone into a tank which holds said solution and which has over said solution a gaseous atmosphere comprising ozone;
(C) applying said aqueous solution of ozone from said tank onto a surface to be sanitized; and
(D) recovering water from said surface and recycling it to said source.

2. A method according to claim 1 wherein said source of water is a storage vessel.

3. A method according to claim 2 wherein said storage vessel comprises means for sensing the pH of the water in the vessel and means for adjusting the pH of said water by adding acidic or alkaline material to the water in response to the sensed pH being above 7.5 or below 6.5.

4. A method according to claim 1 wherein the solution manufactured by said step of injecting said gaseous stream of ozone directly into said stream of water comprises 1% to 15% (w/w) ozone.

5. A method according to claim 1 wherein said tank which holds said ozone solution comprises a vent for venting gas from the gaseous atmosphere over said solution.

6. A method according to claim 5 wherein said vent further comprises an ozone destroyer.

7. A method according to claim 1 wherein the ozone concentration in the solution in said tank is 1 to 10 ppm.

## Patentansprüche

1. Verfahren zur Hygienisierung, wobei im Zuge des Verfahrens
(A) eine wässerige Lösung von Ozon hergestellt wird, indem
(A.1) ein gasförmiger Ozonstrom aus Sauerstoff mit einer Reinheit von mindestens 90 Volumenprozent erzeugt wird
(A.2) ein Wasserstrom mit einem pH-Wert von 6,5 bis 7,5 von einer Quelle bereitgestellt wird, welche den pH-Wert des Wassers überwacht und den pH-Wert falls nötig einstellt, um den pH-Wert in dem besagten Bereich zu halten, und
(A.3) der gasförmige Ozonstrom direkt in den Wasserstrom eingebracht wird;
(B) die wässerige Ozonlösung in einen Tank eingebracht wird, welcher die Lösung enthält und welcher über der Lösung eine gasförmige Atmosphäre, die Ozon enthält, aufweist;
(C) die wässerige Ozonlösung von dem Tank auf eine zu hygienisierende Oberfläche aufgebracht wird; und
(D) von der Oberfläche Wasser gewonnen und der Quelle rückgeführt wird.

2. Verfahren gemäß Anspruch 1, bei welchem die Wasserquelle ein Speicherbehälter ist.

3. Verfahren gemäß Anspruch 2, bei welchem der Speicherbehälter Mittel zum Erfassen des pH-Werts des Wassers in dem Behälter sowie Mittel zum Einstellen des pH-Werts des Wassers durch Zusatz von sauren oder basischen Materialien zu dem Wasser in Ansprechen darauf, dass der erfasste pH-Wert über 7,5 oder unter 6,5 liegt, aufweist.

4. Verfahren gemäß Anspruch 1, bei welchem die durch den Schritt des direkten Einbringens des gasförmigen Ozonstroms in den Wasserstrom erzeugte Lösung 1 % bis 15 % (Gewicht: Gewicht) Ozon aufweist.

5. Verfahren gemäß Anspruch 1, bei welchem der Tank, der die Ozonlösung enthält, eine Entlüftung aufweist, um Gas von der gasförmigen Atmosphäre über der Lösung zu entlüften.

6. Verfahren gemäß Anspruch 5, bei welchem der Entlüfter ferner einen Ozonvernichter aufweist.

7. Verfahren gemäß Anspruch 1, bei welchem die Ozonkonzentration in der Lösung in dem Tank 1 bis 10 ppm beträgt.

## Revendications

1. Procédé de stérilisation, comprenant
(A) la préparation d'une solution aqueuse d'ozone en
(A.1) fabriquant un courant gazeux d'ozone à partir d'oxygène ayant une pureté d'au moins 90 % en volume,
(A.2) se procurant un courant d'eau ayant un pH de 6,5 à 7,5 à partir d'une source qui contrôle le pH de ladite eau et ajuste le pH comme nécessaire pour maintenir le pH dans ladite plage, et
(A.3) injectant ledit courant gazeux d'ozone directement dans ledit courant d'eau ;
(B) l'introduction de ladite solution aqueuse d'ozone dans une cuve qui contient ladite solution et qui présente, sur ladite solution, une atmosphère gazeuse comprenant de l'ozone ;
(C) l'application de ladite solution aqueuse d'ozone depuis ladite cuve sur une surface devant être stérilisée ; et
(D) la récupération d'eau à partir de ladite surface et son recyclage à ladite source.

2. Procédé selon la revendication 1, dans lequel ladite source d'eau est un réservoir de stockage.

3. Procédé selon la revendication 2, dans lequel ledit réservoir de stockage comprend un moyen destiné à capter le pH de l'eau dans le réservoir et un moyen destiné à ajuster le pH de ladite eau par l'addition d'une matière acide ou alcaline à l'eau en réponse au fait que le pH capté est supérieur à 7,5 ou inférieur à 6,5.

4. Procédé selon la revendication 1, dans lequel la solution fabriquée par ladite étape d'injection dudit courant gazeux d'ozone directement dans ledit courant d'eau comprend 1 % à 15 % (en poids/poids) d'ozone.

5. Procédé selon la revendication 1, dans lequel ladite cuve qui contient ladite solution d'ozone comporte un évent pour évacuer le gaz provenant de l'atmosphère gazeuse au-dessus de ladite solution.

6. Procédé selon la revendication 5, dans lequel ledit évent comporte en outre un dispositif de destruction de l'ozone.

7. Procédé selon la revendication 1, dans lequel la concentration d'ozone dans la solution dans ladite cuve est de 1 à 10 ppm.
